Europäisches Patentamt

European Patent Office

Office européen des brevets

(11) Veröffentlichungsnummer: **0 305 803**
**A2**

## (12) EUROPÄISCHE PATENTANMELDUNG

(21) Anmeldenummer: 88113318.5

(22) Anmeldetag: 17.08.88

(51) Int. Cl.⁴: **G01N 33/49** , //G01N33/52, **G01N33/543,G01N33/80**

Patentansprüche für folgenden Vertragsstaat: ES.

(30) Priorität: 31.08.87 DE 3729001

(43) Veröffentlichungstag der Anmeldung:
08.03.89 Patentblatt 89/10

(84) Benannte Vertragsstaaten:
AT BE CH DE ES FR GB IT LI LU NL SE

(71) Anmelder: **BEHRINGWERKE**
**Aktiengesellschaft**
**Postfach 1140**
**D-3550 Marburg 1(DE)**

(72) Erfinder: **Friesen, Heinz-Jürgen Dr.**
**Im Dorfe 4**
**D-3550 Marburg(DE)**
Erfinder: **Hachmann, Henning, Dr.**
**Völklinger Weg 66**
**D-6000 Frankfurt am Main 71(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) Vorrichtung und Verfahren zur Abtrennung von Blutzellen aus Körperflüssigkeiten, die Erythrozyten enthalten, und deren Verwendung.

(57) Es wird eine Vorrichtung und ein Verfahren zur Abtrennung von Blutzellen aus erythrozytenhaltigen Körperflüssigkeiten beschrieben. Die Vorrichtung besteht aus einer faserhaltigen Filterschicht, die Erythrozyten agglutinierende Antikörper und gegebenenfalls ein Lektin enthält, oder aus einer Filterschicht aus Glasfasern und einem Lektin. Sie wird hergestellt durch Tränken der Filterschicht mit einer Lösung des Antikörpers und/oder des Lektins und anschließender Trocknung. Die Vorrichtung wird in diagnostischen Elementen verwendet.

EP 0 305 803 A2

## Vorrichtung und Verfahren zur Abtrennung von Blutzellen aus Körperflüssigkeiten, die Erythrozyten enthalten, und deren Verwendung

Die Erfindung betrifft Filter und deren Verwendung und Verfahren zur Abtrennung von Blutzellen aus Körperflüssigkeiten, die Erythrozyten enthalten.

In klinisch-chemischen Untersuchungen sind Plasma oder Serum, die aus Vollblut gewonnen werden, das bedeutendste Probenmaterial. Der direkte Einsatz von Vollblut verbietet sich oft, da insbesondere die Erythrozyten durch ihre Eigenfarbe oder als Partikel die Tests stören, Deshalb werden Blutzellen durch Zentrifugation entfernt. Bei der Durchführung vieler verschiedener Analysen aus einer Blutprobe ist dies die Methode der Wahl. In der Notfalldiagnostik oder bei Einzeltests sind schnellere Verfahren zur Gewinnung von Serum oder Plasma sowie Testmethoden, die mit einem kleineren Blutvolumen auskommen, gefragt.

Aus EP-A-O 143 574 ist bekannt, Erythrozyten aus Vollblut zu entfernen, indem erythrozyten-agglutinierende Substanzen wie Antikörper gegen Erythrozyten oder Lektine in Lösung zugesetzt werden und das Agglutinat abgetrennt wird.

Die EP-A-O 045 476 beschreibt die Verwendung von Glasfaserfiltern zur Gewinnung von Plasma oder Serum aus Vollblut.

Die DE-35 08 427 beschreibt die Gewinnung von Plasma oder Serum aus Vollblut, mittels einer mit einem Lektin imprägnierten saugfähigen Matrix.

Die EP-A-0 223 978 beschreibt Vorrichtungen und Methoden zur Blutgruppenbestimmung unter Verwendung von porösen Materialien, an die im wesentlichen reine Antikörper, gerichtet gegen antigene Determinaten der Blutgruppen von Erythrozyten, gebunden sind.

Es wurde gefunden, daß Filterelemente nach Tränken mit einer Lösung von Antikörpern und gegebenenfalls Lektinen, die in der Lage sind, Erythrozyten zu agglutinieren, und nach Trocknen der Elemente, sämtliches Blutgewebe zurückhalten, wenn Vollblut oder eine Erythrozyten enthaltende Körperflüssigkeit aufgetragen wird. Dabei wird zellfreies Plasma, Serum oder Liquor als Filtrat erhalten. Glasfaserhaltige Filter, die mit einer Lösung von Erythrozyten agglutinierendem Lektin getränkt und dann getrocknet wurden, zeigten ebenfalls eine gute Trennung der zellfreien Körperflüssigkeit von dem Blutgewebe.

Gegenstand der Erfindung ist daher eine Vorrichtung zur Abtrennung von Blutzellen aus einer Körperflüssigkeit mittels einer faserhaltigen Filterschicht, dadurch gekennzeichnet, daß die Filterschicht Antikörper, die alle Erythrozyten agglutinieren, und gegebenenfalls ein Lektin enthält.

Gegenstand der Erfindung ist auch eine Vorrichtung zum Abtrennen von Blutzellen aus einer Körperflüssigkeit mittels einer faserhaltigen Filterschicht, dadurch gekennzeichnet, daß die Filterschicht Glasfasern und ein Lektin enthält.

Gegenstand der Erfindung ist außerdem ein Verfahren zur Herstellung einer Vorrichtung mindestens bestehend aus einer faserhaltigen Filterschicht, welche Erythrozyten agglutinierende Antikörper und gegebenenfalls ein Lektin enthält, dadurch gekennzeichnet, daß man die Filterschicht mit einer wässrigen Lösung des Antikörpers und gegebenenfalls des Lektins tränkt und die getränkte Filterschicht trocknet.

Gegenstand der Erfindung ist weiterhin ein Verfahren zur Herstellung einer Vorrichtung enthaltend eine glasfaserhaltige Filterschicht und ein Lektin, dadurch gekennzeichnet, daß man die Filterschicht mit einer wässrigen Lösung eines Lektins tränkt und die getränkte Filterschicht trocknet.

Gegenstand der Erfindung ist weiterhin ein Verfahren zur Gewinnung von gewebefreier Körperflüssigkeit, indem man eine erythrozytenhaltige Körperflüssigkeit auf eine solche Vorrichtung bringt und die gewebefreie Körperflüssigkeit als Filtrat ablaufen oder von einem saugfähigen Material, welches mit der Vorrichtung in Verbindung steht, aufsaugen läßt.

Gegenstand der Erfindung ist außerdem die Verwendung einer solchen Vorrichtung in einem diagnostischen Element zum Nachweis und zur Bestimmung eines in der gewebefreien Körperflüssigkeit enthaltenen Bestandteils.

Geeignete Materialien für Bestandteile einer Antikörper enthaltenden Vorrichtung sind Zellulosefaser, chemisch derivatisierte Zellulosefaser, Glas- oder Quarzfaser oder andere mineralische Faser, natürliche Polymerfaser, deren Basisbestandteile andere als Zellulose sind. Die genannten Fasern können als einzelne Faser oder als Kombination den fasrigen Bestandteil der Filterschicht bilden, wobei sowohl eine gewobene als auch eine nicht gewobene, d.h. vliesartige Struktur geeignet ist.

Als Antikörper sind solche geeignet, die Erythrozyten agglutinieren. Sie werden vorzugsweise durch Immunisierung von Tieren mit einem Gemisch von Erythrozyten erhalten. Es können mono- oder polyklonale Antikörper verwendet werden.

Lektine, die gegebenenfalls der Imprägnierlösung zugesetzt werden, sind solche, die ebenfalls Erythrozyten agglutinieren, oder solche, die mit dem Antikörper reagieren.

Die Antikörper und gegebenenfalls die Lektine werden vorzugsweise auf die faserhaltige Filterschicht direkt aus wässriger gepufferter oder ungepufferter Lösung aufgetrocknet.

Die Konzentrationen an Antikörpern und gegebenenfalls Lektinen in dieser auch als Imprägnierlösung zu bezeichnenden Lösung wird dem gewünschten Erythrozyten-Rückhalteeffekt der faserhaltigen Filterschicht angepaßt. Bei Verwendung von Filtern aus Glasfaser wird eine Konzentration an Antikörper und gegebenenfalls Lektin, die mindestens der 5-fachen Konzentration entspricht, die zur vollständigen Agglutination ausreicht, bevorzugt.

Besteht die Vorrichtung aus einem Filter mit Glasfaser und Lektin, aber keinen Antikörpern als Bestandteilen, wird zu ihrer Herstellung ein glasfaser-haltiges Filter mit einer Lösung eines oder mehrerer Lektine getränkt und danach getrocknet. Dabei wird eine Lösung eines Lektins oder eines Gemischs von Lektinen verwandt, die alle menschlichen Erythrozyten agglutinieren. Bevorzugt werden die Lektine von Solanum tuberosum (Kartoffel) oder von Lycopesicon esculantum (Tomate) als einziges Lektin oder in Kombination mit anderen Lektinen. Das Lektin liegt in der Imprägnierlösung in einer bevorzugten Konzentration von 1-20 mg/l vor. Besonders bevorzugt werden 5 mg/l.

Den Imprägnierlösungen können noch Proteine und Detergentien zugesetzt werden, die das Binden von Analyt oder Antikörpern an die Oberfläche limitieren. Diese Zusätze werden in ihren Konzentrationen dabei so gewählt, daß eine Inhibition der Haemagglutination vermieden wird. Die Detergentien werden in ihrer Konzentration ferner so limitiert, daß sie keine Hämolyse bewirken. Die minimale Detergenzkonzentration wird so eingestellt, daß für den Test eine optimale Benetzbarkeit des Filters gewährleistet ist.

Geeignete Proteine sind ®Polygelin, Serumalbumin, Gelatine, Ovalbumin oder Casein. Der bevorzugte Konzentrationsbereich liegt dabei bei 0,5 - 10 g/l, besonders bevorzugt bei 0,8 - 1,2 g/l.

Als Detergentien sind ®Zwittergent 3-10 (Calbiochem), ®Dapral 205 (AKZO) oder ®Pluronic F-68 (Atlas Chemie) in einer Konzentration von 0,1 - 5 g/l und besonders von 0,1 - 0,2 g/l geeignet. Bevorzugt geeignet sind ®Zwittergent 3-08, ®Dapral 205 in einer Konzentration von 0,1 - 5 g/l, wobei 0,1 - 0,2 g/l bevorzugt wird.

Der pH-Wert der Imprägnierlösung liegt bevorzugt im Bereich von pH 6 - 8, besonders bevorzugt bei pH 7,0 -7,5.

Ist die Vorrichtung in einem schichtförmig oder flächenförmig angeordneten diagnostischen Element enthalten, wobei sie durch eine Fläche mit einer saugfähigen Schicht in Verbindung steht, wird nach Aufgabe von erythrozytenhaltiger Körperflüssigkeit auf eine andere Fläche der Vorrichtung, die nicht mit einer saugfähigen Schicht verbunden ist, die gewebefreie Flüssigkeit von der saugfähigen Schicht durch Kapillarkräfte aufgesaugt und steht somit zum Nachweis und zur Bestimmung einer in ihr enthaltenen Komponente zur Verfügung.

Beispiele

Herstellung von Erythrozyten-agglutinierenden Antikörpern

Kaninchen wurden mit einem Pool von gewaschenen Erythrozyten von 30 Spendern, bei denen die vier Blutgruppen 0, A, B und AB anteilmäßig gleich vorhanden waren, immunisiert. Agglutinierende Antikörper wurden in einer Verdünnungsreihe des Serums in phosphat-gepufferter Kochsalzlösung (PBS) gemessen, indem 50 $\mu$l Serumverdünnung, 50 $\mu$l PBS und 25 $\mu$l 20ml/l Erythrozytensuspension in PBS in Vertiefungen von Mikrotestplatten 60 min bei 37°C inkubiert, Teppich- und Knopfbildung nach einigen Stunden abgelesen und die Verdünnungsstufe, bei der der Übergang von Teppich- zur Knopfbildung zu sehen war, als Titer registriert. Mit Erythrozyten der Blutgruppen 0, A, B und AB wurden Titer zwischen 640 und 1280 erhalten.

Aus dem Serum wurde durch Chromatographie an DEAE-Zellulose die Immunglobulin G (IgG)-Fraktion gewonnen, die nach Einstellung auf 40 mg/l Protein einen agglutinierenden Antikörpertiter von jeweils 2560 mit Erythrozyten der genannten Blutgruppen hatte. Diese Präparation wird im folgenden als Ery-AK bezeichnet.

3

Lösungen von Ery-AK

Es wurden wässrige Lösungen von 5 µg bis 22 g/l Ery-AK, von denen noch 1 g/l ®Polygeline und 0,1 g/l ®Zwittergent zugesetzt worden war, hergestellt und auf pH 7,2 eingestellt.

Lösungen von Lektinen

Lektin von Solanum tuberosum oder von Lycopersicon esculantum wurde in einer wässrigen Lösung von 1 g/l ®Polygeline und 0,1 g/l ®Zwittergent in Konzentrationen von 5 µg/l gelöst und die derart erhaltene Lösung auf pH 7,2 eingestellt.

Faserhaltige Filter

Es wurden 6 x 6 mm oder 7 x 7 mm große Stücke aus
  a) Nr. 2668/8 Papier der Fa. Schleicher & Schüll, Dassel, oder
  b) Nr. 030-PG Glasfaserfilter der Fa. Pall, Dreieich (FRG), bei dem das Trägervlies entfernt worden war, oder
  c) Glasfaserfilter Typ A der Fa. Whatmann oder
  d) Glasfaserfilter Extra Thick der Fa. Gelman oder
  e) Quarzfaserfilter QMA der Fa. Gelman ausgeschnitten.

Herstellung einer erfindungsgemäßen Vorrichtung

a) Vorrichtungen für Verfahren mit Papier als saugfähige Unterlage

Die faserhaltigen Filter wurden mit 8 bzw. 38 bzw. 40 µl/0,36 gcm (entsprechend dem Saugvolumen der jeweiligen Filter) Lösung von Serumalbumin oder Ery-Ak (2, 4, 6, 8, und 22 mg/ml) getränkt und die Filter anschließend 20 min bei 50 °C im Umlufttrockenschrank getrocknet. Das Glasfaserfilter, Typ A wurde mit Antikörperverdünnungen in 10er Stufen von 0,5 g/l bis 50 µg/l mit dem Saugvolumen des Filters imprägniert.

b) Vorrichtungen für Verfahren mit Agarose enthaltendem Film als saugfähiger Schicht

Größere Filterstücke wurden mit den oben angegebenen Imprägnierlösungen getränkt. Überschüsse an Imprägnierlösung wurden mit Filterpapier abgetupft.
Die im Filter verbleibende Flüssigkeitsmenge entsprach 80 - 90 % des Saugvolumens. Die Filter wurden anschließend 6 Stunden bei 30 °C im Vakuumtrockenschrank bei 30 Torr getrocknet.

Verwendung einer erfindungsgemäßen Vorrichtung

Versuchsaufbau:

a) Versuche mit Papier als Plasma-aufsaugendem Substrat:

6 X 6 mm große Vorrichtungen, hergestellt durch Imprägnieren der beschriebenen faserhaltigen Filter mit Ery-AK wurden an einem Ende auf Filterpapier Nr. 2040a der Fa. Schleicher & Schüll, Dassel, aufgelegt. Das Papier 2040a war mit doppelseitigem Tesaklebeband der Fa. Beiersdorf auf einer Unterlage fixiert. Für Vergleichszwecke wurden auch nicht imprägnierte faserhaltige Filter auf das Nr. 2040a Papier aufgelegt.

b) Beispiele mit Agarose enthaltender Filmschicht

7 x 7 mm große Vorrichtungen, hergestellt durch Imprägnieren der beschriebenen faserhaltigen Filter mit Ery-AK und ein 7 x 11 mm großes Stück Polystyrolfilm, das mit 18 g Agarose und 6 g Titandioxyd/ qm (Saugvolumen: 7 μl) beschichtet waren, wurden Seite an Seite auf eine 7 x 20 mm großes Zelluloseacetatpropionat-Unterlage aufgelegt, die auf ihrer Oberfläche in Längsrichtung 20 convexe Rillen von 0,125 mm Tiefe aufwies. Die mit Agarose beschichtete Seite des Films war dabei in Kontakt mit der Rillen-tragenden Seite der Unterlage.

Versuchsdurchführung:

a) Aufsaugen von Plasma in Papier

Auf die Vorrichtung mit faserhaltigem Filter Nr. 2668/8 wurden je 50 μl Heparinblut und auf die Vorrichtung mit Glasfaserfilter Nr. 030-PG wurden je 15 μl Heparinblut aufgetropft. Die Diffusion der Flüssigkeit durch die Vorrichtungen in das darunterliegende Papier Nr. 2040a wurde beobachtet.

b) Aufsaugen von Plasma in Filmschicht

Hier wurden 65 μl Heparinblut auf die Vorrichtungen aufgetropft. Es wurden die Zeiten für das Füllen der mit dem Film abgedeckten Rillen sowie die Erythrozytenkontamination des Plasmas registriert.

Ergebnisse:

a) Versuche mit Filterpapier enthaltender Vorrichtung, Papier als Plasma-aufsaugendes Mittel und Blut der Blutgruppe B:

Beim nicht oder mit Serumalbumin imprägnierten Filterpapier wanderte stark rotgefärbtes, Erythrozyten enthaltendes Filtrat in das untenliegende Mittel. Die Rotfärbung wanderte mit der Flüssigkeitsfront. Bei den erfindungsgemäßen Vorrichtungen wanderte die Rotfärbung maximal halb so weit wie die Flüssigkeitsfront. Bei den Vorrichtungen, die mit Lösungen von 8 und 22 g/l Ery-AK imprägniert worden waren, trat keine Rotfärbung aus der Vorrichtung aus.

b) Versuche mit Vorrichtungen enthaltend Glasfaserfilter 030 PG, Papier als Plasma-aufsaugendes Mittel und Blut der Blutgruppe B:

Beim nicht vorimprägnierten Glasfaserfilter wanderte eine starke Rotfärbung aus dem Filter mit der Flüssigkeitsfront. Beim mit Serumalbumin imprägnierten Filter wanderte die Rotfärbung ca. 2/3 der Strecke der Front aus dem Filter heraus. Bei den mit Ery-Ak imprägnierten Filtern, also den erfindungsgemäßen Vorrichtungen, trat in allen Fällen keine Rotfärbung mehr aus dem Filter aus. Die vom darunterliegende Filter aufgesaugte Flüssigkeit hat die Farbe eines nicht hämolytischen Serums oder Plasmas.

c) Versuche mit Glasfaserfilter GF/B enthaltender Vorrichtung, Papier als Plasma-aufsaugendes Mittel und Blut unbekannter Blutgruppe:

Beim nicht vorimprägnierten Filter wanderte ein roter Schleier bei Aufgabe von 70 µl Vollblut auf das Filter aus diesem heraus.

Bei Vorimprägnierungen bis zu Antikörperkonzentrationen von 5 mg/l herunter war dieser Schleier nicht zu beobachten.

Bei Vorimprägnierungn mit Lektin aus Solanum tuberosum oder aus Lycopersicon esculantum in Konzentrationen bis herunter auf 5 mg/l war dieser Schleier ebenfalls nicht zu beobachten.

d) Versuche mit Vorrichtungen enthaltend Glasfaserfilter Extra Thick oder Quarzfaserfilter QMA, Agarosefilm als Plasma-aufsaugendes Mittel und Blut verschiedener Blutgruppen:

Bei vollständiger Abtrennung der Erythrozyten in der Vorrichtung waren keine Erythrozyten in den Rillen vor dem weißen Hintergrund des Films zu erkennen (Testergebnis: + bis + +). Bei schlechten Testergebnissen ( - ) waren zumindest einzelne Rillen, die mit Erythrozyten kontaminiertes Plasma enthielten, als rote Streifen zu erkennen. Wurden Quarz- oder Glasfaser enthaltende Vorrichtungen verwendet, die mit Lösungen von Ery-AK in einer Konzentration größer als 0,1 g/l oder mit Lektin in einer Konzentration größer als 5 mg/l imprägniert worden waren, war das Testergebnis gut.

Das Testergebnis ist im Folgenden zusammengefaßt.

| | | Testergebnis | | |
|---|---|---|---|---|
| Filtermaterial | Zeit bis zum Füllen des Testelementes m. Plasma | ohne Imprägnierg. | mit Ery-AK | mit Lektin |
| Quarzfaser | 96 - 102 sec (n = 4) | + | + + | + + |
| Glasfaser | 15 - 41 sec (n = 4) | - | + + | + + |

Verwendung der Vorrichtung in einem diagnostischen Element zur Bestimmung von Glucose in Vollblut

Zur Herstellung eines Mittels zur Bestimmung von Glucose in Vollblut wurde das Rapignost® Glucosetestfeld des Uringlucose-Teststreifens der Behringwerke in einer Abmessung von 6 x 15 mm mit doppelseitigem Tesaklebeband der Fa. Beiersdorf auf einer Unterlage fixiert. Ein mit Anti-Ery in einer Konzentration von 2 mg/ml imprägniertes Pall Glasfaserfilter 030 PG (Abmessung: 6 x 6 mm), bei dem wie zuvor das Trägervlies entfernt worden war, wurde am einen Ende auf das RAPIGNOST-Testpapier aufgelegt. Auf das Glasfaserfilter wurden je 15 µl Vollblut mit unterschiedlichem Glucosegehalt aufgetropft. Die sich bildende Färbung im Testpapier wurde beobachtet. Innerhalb von 1 -2 min bildete sich im vom mit dem aus dem Filter austretenden Plasma benetzten Testpapier eine Blaugrünfärbung. Diese entsprach in ihrer Intensität derjenigen, die man erhielt, wenn anstelle des Vollblutes abzentrifugiertes Plasma oder Serum, gewonnen aus den gleichen Blutproben, eingesetzt wurde. Gleiche Ergebnisse erhielt man, wenn entsprechend imprägniertes Gelman Typ A Glasfaserfilter eingesetzt wurde. Beim 2668/8-Papier konnte in dem Bereich des Testpapiers abgelesen werden, in dem das Plasma von den Erythrozyten abgetrennt war.

## Ansprüche

1. Vorrichtung zum Abtrennen von Blutzellen aus Körperflüssigkeit mittels einer faserhaltigen Filterschicht, dadurch gekennzeichnet, daß die Filterschicht Antikörper, die alle Erythrozyten agglutinieren, und gegebenenfalls ein Lektin enthält.

2. Vorrichtung zum Abtrennen von Blutzellen aus Körperflüssigkeit mittels einer faserhaltigen Filterschicht, dadurch gekennzeichnet, daß die Filterschicht Glasfasern und ein Lektin enthält.

3. Vorrichtung nach Anspruch 1, dadurch gekennzeichnet, daß die Filterschicht natürliche Polymerfaser vorzugsweise Zellulosefaser, chemisch derivatisierte Zellulosefaser, Glasfaser oder andere mineralische Faser, synthetische Polymerfaser als einzigen Faserbestandteil oder eine Mischung mehrerer Faserbestandteile enthält.

4. Vorrichtung nach Anspruch 1, dadurch gekennzeichnet, daß der Antikörper polyklonal ist.

5. Vorrichtung nach Anspruch 1, dadurch gekennzeichnet, daß der Antikörper ein monoklonaler und gegebenenfalls ein polyklonaler Antikörper ist.

6. Verfahren zur Herstellung einer Vorrichtung bestehend aus einer faserhaltigen Filterschicht, welche Erythrozyten agglutinierende Antikörper und gegebenenfalls Lektine enthält, dadurch gekennzeichnet, daß man die Filterschicht mit einer wässrigen Lösung des Antikörpers und gegebenenfalls des Lektins tränkt und die getränkte Filterschicht trocknet.

7. Verfahren zur Herstellung einer Vorrichtung bestehend aus einer glasfaserhaltigen Filterschicht, welche Lektine enthält, dadurch gekennzeichnet, daß man die Filterschicht mit einer wässrigen Lösung des Lektins tränkt und die getränkte Filterschicht trocknet.

8. Verfahren zur Gewinnung von gewebsfreier Körperflüssigkeit, in dem man erythrozytenhaltige Körperflüssigkeit auf eine Vorrichtung gemäß Anspruch 1 oder 2 aufbringt und die gewebefreie Körperflüssigkeit als Filtrat gewinnt.

9. Verfahren zur Gewinnung von gewebefreier Körperflüssigkeit, in dem man erythrozytenhaltige Körperflüssigkeit mit einer Vorrichtung gemäß Anspruch 1 oder 2 in Kontakt bringt und die gewebsfreie Körperflüssigkeit in ein saugfähiges Material, welches mit der Vorrichtung in Verbindung steht, aufsaugen läßt.

10. Verwendung einer Vorrichtung nach Anspruch 1 oder 2 in einem diagnostischen Element zum Nachweis und zur Bestimmung eines in der zellfreien Körperflüssigkeit enthaltenen Bestandteils.

Patentansprüche für den folgenden Vertragsstaat: ES

1. Verfahren zur Herstellung einer Vorrichtung bestehend aus einer faserhaltigen Filterschicht, welche Erythrozyten agglutinierende Antikörper und gegebenenfalls Lektine enthält, dadurch gekennzeichnet, daß man die Filterschicht mit einer wässrigen Lösung des Antikörpers und gegebenenfalls des Lektins tränkt und die getränkte Filterschicht trocknet.

2. Verfahren zur Herstellung einer Vorrichtung bestehend aus einer glasfaserhaltigen Filterschicht, welche Lektine enthält, dadurch gekennzeichnet, daß man die Filterschicht mit einer wässrigen Lösung des Lektins tränkt und die getränkte Filterschicht trocknet.

3. Verfahren zur Gewinnung von gewebsfreier Körperflüssigkeit, in dem man erythrozytenhaltige Körperflüssigkeit auf eine faserhaltige Filterschicht aufbringt, die Antikörper, die alle Erythrozyten agglutinieren, und gegebenenfalls ein Lektin enthält, und die gewebefreie Körperflüssigkeit als Filtrat gewinnt.

4. Verfahren zur Gewinnung von gewebefreier Körperflüssigkeit, in dem man erythrozytenhaltige Körperflüssigkeit auf eine faserhaltige Filterschicht aufbringt, die Glasfasern und ein Lektin enthält, und die gewebefreie Körperflüssigkeit als Filtrat gewinnt.

5. Verfahren zur Gewinnung von gewebefreier Körperflüssigkeit, in dem man erythrozytenhaltige Körperflüssigkeit mit einer faserhaltigen Filterschicht in Kontakt bringt, die Antikörper, die alle Erythrozyten agglutinieren, und gegebenenfalls ein Lektin enthält, und die gewebsfreie Körperflüssigkeit in ein saugfähiges Material, welches mit der Vorrichtung in Verbindung steht, aufsaugen läßt.

6. Verfahren zur Gewinnung von gewebefreier Körperflüssigkeit, in dem man erythrozytenhaltige Körperflüssigkeit mit einer faserhaltigen Filterschicht in Kontakt bringt, die Glasfasern und ein Lektin enthält, und die gewebsfreie Körperflüssigkeit in ein saugfähiges Material, welches mit der Vorrichtung in Verbindung steht, aufsaugen läßt.